Europäisches Patentamt

European Patent Office

Office européen des brevets

(11) Veröffentlichungsnummer: **0 249 823 B1**

# EUROPÄISCHE PATENTSCHRIFT

(45) Veröffentlichungstag der Patentschrift: **18.12.91**

(51) Int. Cl.⁵: **A61B 5/05**, A61N 1/365

(21) Anmeldenummer: **87108116.2**

(22) Anmeldetag: **04.06.87**

(54) **Vorrichtung zur Steuerung eines Herzschrittmachers mittels Impedanzmessung an Körpergeweben.**

(30) Priorität: **16.06.86 DE 3620280**

(43) Veröffentlichungstag der Anmeldung:
**23.12.87 Patentblatt 87/52**

(45) Bekanntmachung des Hinweises auf die
Patenterteilung:
**18.12.91 Patentblatt 91/51**

(84) Benannte Vertragsstaaten:
**DE FR GB IT NL SE**

(56) Entgegenhaltungen:
**EP-A- 0 089 014**
**GB-A- 2 070 282**
**US-A- 3 532 086**
**US-A- 4 030 485**

**MEDICAL & BIOLOGICAL ENGINEERING & COMPUTING, Band 20, Nr. 3, Mai 1982, Seiten 293-298, IFMBE, Stevenage, GB; A.J. WILSON et al.: "Methods of filtering the heart-beat artefact from the breathing waveform of infants obtained by impedance pneumography"**

(73) Patentinhaber: **SIEMENS AKTIENGESELL-SCHAFT**
**Wittelsbacherplatz 2**
**W-8000 München 2(DE)**

(72) Erfinder: **Heinze, Roland**
**Simbacher Strasse9**
**W-8000 München 80(DE)**
Erfinder: **Stangl, Karl, Dr.**
**Landwehrstrasse 7**
**W-8000 München 2(DE)**

**Beschreibung**

Die Erfindung bezieht sich auf eine Vorrichtung zur Impedanzmessung an Körpergeweben gemäß Oberbegriff des Patentanspruchs 1.

Eine solche Vorrichtung mit einer Auswerteeinrichtung im Sinne der Heraustrennung sowohl von niederfrequenten als auch höherfrequenten signalanteilen ist im Zusammenhang mit der Bestimmung von Blutverlusten bei einer Operation durch die US-PS 3 532 086 vorbekannt. Der niederfrequente Anteil ist ein Maß für das Blutvolumen.

Impedanzmessungen im Körpergewebe (einschließlich Blut) dienten bisher zur Bestimmung von mechanischen Volumenänderungen des Körpers, z.B. des Schlagvolumens des Herzens sowie auch der Atmung aufgrund Thoraxbewegungen. Die Änderung der Impedanz kann zur Frequenssteuerung von Herzschrittmachern verwendet werden. So ist aus der US-PS 4 303 075 eine Vorrichtung zur Impedanzmessung bei einem frequenzgesteuerten Herzschrittmacher bekannt. Dort werden jedoch lediglich die höherfrequenten Signalanteile als Maß für die Atmung oder das Schlagvolumen des Herzens aus dem erfaßten Impedanzsignal herausgefiltert und damit die Stimulationsfrequenz gesteuert.

Grundlage der Impedanzmessung ist vereinfacht die folgende physikalische Beziehung:

$$R = \frac{l}{\sigma_R \cdot F}$$

wobei R die Impedanz,
$\sigma_R$ der Leitwert

$$\left( \frac{1}{\Omega \cdot cm} \right),$$

1 der wirksame Elektrodenabstand (cm) und
F der wirksame Leitungsquerschnitt (cm²)
zwischen den Elektroden ist.

Die Messung periodischer Impedanzschwankungen erfaßt also die Änderungen von 1 oder F auf der Leitungsstrecke.

Eine unmittelbare Aussage über den Stoffwechsel, der ein direktes Maß für die Körperbelastung wäre, ist nicht möglich.

Aufgabe der vorliegenden Erfindung ist es, eine Vorrichtung zur Impedanzmessung der eingangs genannten Art im Sinne einer stoffwechselabhängigen Herzschrittmachersteuerung weiterzubilden.

Die Aufgabe wird erfindungsgemäß durch die kennzeichnenden Merkmale des Patentanspruchs 1 gelöst.

Die Erfindung geht aus von der Erkenntnis, daß im Leitwert $\sigma_R$ der obengenannten Beziehung metabolische Anteile stecken, die ein direktes Maß für den Stoffwechsel (damit direkter Bezug zur Belastung) abgeben. Sie geht ferner davon aus. daß im erfaßten Impedanzsignal höherfrequente Signalanteile stecken, die im wesentlichen Änderungen von 1 oder F wiederspiegeln, und daß auch ein niederfrequenter Anteil vorhanden ist, der ein direktes Maß für den Leitwert $\sigma_R$ ist (im Gegensatz zum Gegenstand der US-PS 3 532 086, der niederfrequente Anteile als Blutvolumen anzeigt). Konventionelle Vorrichtungen zur Impedanzmessung in der Herzschrittmachertechnik, wie z.B. jene der US-PS 4 303 075, filtern die höherfrequenten Signalanteile als Maß für die Atmung oder das Schlagvolumen des Herzens aus dem erfaßten Impedanzsignal heraus und steuern damit z.B. die Frequenz eines Herzschrittmachers. Bei vorliegender Erfindung werden jedoch anstelle der höherfrequenten die niederfrequenten Signalanteile des Impedanzsignals abgetrennt und zur Frequenzsteuerung eines Herzschrittmachers herangezogen. Man erhält dadurch ein direkt vom Leitwert abhängiges Signal, das eine unmittelbare Aussage über den Stoffwechsel und damit die momentane Belastung, z.B. eines Patienten, zuläßt und somit ein erheblich genaueres Steuerungssignal für die Stimulationsfrequenz als der bekannte höherfrequente Anteil des Impedanzsignals bildet.

In einer vorteilhaften Ausgestaltung der Erfindung ist die Grenzfrequenz des Tiefpaßfilters in Abhängigkeit von der Stimulationsfrequenz variierbar, in dem Sinne, daß sie mit steigender Stimulationsfrequenz zu höheren Werten und mit sinkender Stimulationsfrequenz entsprechend zu niedrigeren Werten verschoben wird. Dadurch ergibt sich der Vorteil, daß die Grenzfrequenz des Filters in Abhängigkeit von der Atemfrequenz, die sich analog zur Belastung (und damit auch zur sich ändernden Stimulationsfrequenz) verändert, immer möglichst hoch ist, aber dennoch gleichzeitig immer unterhalb der Atemfrequenz liegt. Dadurch kann es nicht zu störendem Einfluß der Atmung auf das Meßergebnis kommen.

In einer weiteren vorteilhaften Ausgestaltung der Erfindung ist zusätzlich noch eine Temperatureinfluß-Kompensationseinrichtung vorgesehen, mit deren Hilfe der gemessene Leitwert von Temperatureinflüssen bereinigt wird.

Weitere Vorteile und Einzelheiten der Erfindung ergeben sich aus der nachfolgenden Beschreibung eines Ausführungsbeispiels anhand der Zeichnung und in Verbindung mit den Unteransprüchen.

Es zeigen:

Figur 1     eine erste und

Figur 2     eine zweite Ausführungsform für die Impedanzmessung als Teile eines in

Figur 3 gezeigten Ausführungsbeispiels der erfindungsgemäßen Vorrichtung in Form eines frequenzgesteuerten Herzschrittmachers.

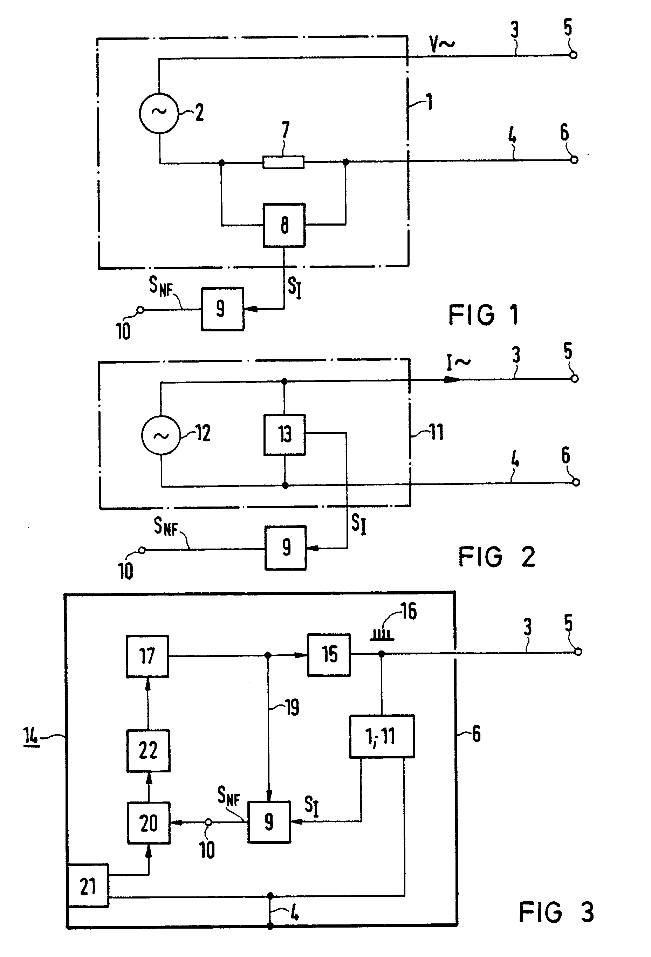

Der Impedanzmesser 1 der Figur 1 umfaßt als Signalquelle zum Einprägen eines elektrischen Signals einen Wechselspannungserzeuger 2 (z.B. 1 kHz-Wechselspannungserzeuger), der über Elektrodenleitungen 3 und 4 mit zugehörigen Elektroden 5 und 6 einem (nicht dargestellten) Körpergewebe eine konstante Wechselspannung V~ (z.B. 1 kHz-Wechsel-spannung) einprägt. Die Meßanordnung der Figur 1 ist dabei vorzugsweise zur intrakorporalen Messung ausgelegt. Es sind also zumindest die Elektroden 5 und 6 im Körpergewebe implantiert. In Abhängigkeit von der eingeprägten Wechselspannung V~ wird über einen niederohmigen Serienwiderstand 7 (z.B. 100 Ohm) der vom Strom in den Elektrodenleitungen 3 und 4 verursachte spannungsabfall mittels eines Spannungsmessers 8 erfaßt. Das Ausgangssignal des Spannungsmessers 8 wird dann einem Tiefpaßfilter 9 zugeführt, dessen obere Grenzfrequenz im Bereich 0,1 bis 0,4 Hz liegt (vorzugsweise in diesem Frequenzbereich variierbar ist, wie später anhand der Figur 3 noch näher erläutert wird). Das Tiefpaßfilter 9 trennt aus dem Ausgangssignal $S_I$ (Impedanzsignal) des Spannungsmessers 8 lediglich die niederfrequenten Signalanteile $S_{NF}$, die dem Leitwert $\sigma_R$ im Körpergewebe entsprechen, heraus. Die herausgetrennten niederfrequenten Signalanteile $S_{NF}$ können vom Signalausgang 10 des Tiefpaßfilters 9 abgenommen werden.

In der Figur 2 umfaßt der Impedanzmesser 11 eine Wechselstromquelle 12 (z.B. 1 kHZ-Wechselstromquelle), die dem Körpergewebe über die Elektrodenleitungen 3 und 4 sowie den zugehörigen Elektroden 5 und 6 einen konstanten Wechselstrom I ~ (z.B. 1 kHz-Wechselstrom) einprägt. Das gesamte Meßsystem ist wieder vorzugsweise als intrakorporales Meßsystem ausgelegt. Gemessen wird diesmal die Wechselspannung zwischen den Elektroden 5 und 6 mittels eines parallelgeschalteten Spannungsmessers 13, der einen Dividierer zur Bildung von 1/V ~ beinhaltet. Das Ausgangssignal $S_I$ (Impedanzsignal) des Spannungsmessers 13 wird dann in derselben Weise, wie zuvor im Zusammenhang mit der Figur 1 beschrieben, in einem Tiefpaßfilter 9 ausgewertet.

Die Figur 3 zeigt die Anwendung einer Meßanordnung gemäß der Figur 1 oder Figur 2 in einem frequenzgesteuerten Herzschrittmacher 14. Gleiche Bauelemente sind dabei mit gleichen Bezugsziffern versehen. Die Elektrode 5 ist im vorliegenden Fall gleichzeitig die Stimulationselektrode des Herzschrittmachers 14, während die Elektrode 6 durch das leitende (z.B. metallische) Gehäuse des Herzschrittmachers gebildet ist. Die Elektrodenleitung 3 entspricht dem Stimulationskatheter des Herzschrittmachers.

Der frequenzgesteuerte Herzschrittmacher 14 umfaßt einen Pulsgenerator 15 für die Stimulationsimpulse 16. Die Folgefrequenz der Stimulationsimpulse 16 (Stimulationsfrequenz) ist am Pulsgenerator 15 über ein Frequenzsteuerteil 17 in Abhängigkeit von dem herausgefilterten niederfrequenten Signalanteil $S_{NF}$ am Ausgang 10 des Tiefpaßfilters 9 steuerbar. Die Steuerung erfolgt in dem Sinne, daß bei sich änderndem Leitwert $\sigma_R$ die Stimulationsfrequenz proportional geändert wird. Die Stimulationsfrequenz steigt also, wenn der herausgefilterte niederfrequente Signalanteil $S_{NF}$ (und damit auch der Leitwert $\sigma_R$) zunimmt. Die Stimulationsfrequenz wird in umgekehrter Weise geringer, wenn die Signalanteile $S_{NF}$ (und damit der Leitwert $\sigma_R$ ) abnehmen.

Wie in der Figur 3 angedeutet ist, ist die Grenzfrequenz des Tiefpaßfilters 9 über eine Steuerleitung 19 in Abhängigkeit vom Ausgangssignal des Frequenzsteuerteils 17 steuerbar in dem Sinne, daß sie im Bereich 0,1 bis 0,4 Hz mit steigender Stimulationsfrequenz zu höheren Werten und mit sinkender Stimulationsfrequenz entsprechend zu niedrigeren Werten verschoben wird. Es tritt der bereits eingangs geschilderte Vorteil ein, daß das Meßergebnis trotz erwünscht höchstmöglicher Grenzfrequenz des Tiefpaßfilters 9 von der Atmung unbeeinflußt bleibt.

Ferner ergibt sich aus der Figur 3, daß die Steuerung des Frequenzsteuerteiles 17 vom Signal $S_{NF}$ nicht unmittelbar, sondern unter Zwischenschaltung eines Korrekturgliedes 20 zur Kompensation von Temperatureinflüssen erfolgt. Der Leitwert $\sigma_R$ erhöht sich mit steigender Temperatur. Das Korrekturglied 20 korrigiert das Signal $S_{NF}$ für den Leitwert in umgekehrter Weise. Zum Erfassen der Temperatur dient ein Temperaturfühler 21, der im Gehäuse des Herzschrittmachers oder z.B. außerhalb des Gehäuses angeordnet sein kann. Es versteht sich von selbst, daß auch der Impedanzmesser 1 bzw. 11 sowie das Tiefpaßfilter 9 vorzugsweise im Inneren des Schrittmachergehäuses untergebracht sind.

Anstelle des Temperaturfühlers 21 und des Korrekturgliedes 20 kann aber auch der Stimulationskatheter 3 selbst aus einem den Temperaturgang der Leitfähigkeit kompensierenden Material (z.B. NTC-Widerstandsmaterial) bestehen oder im Stimulationskatheter 3 kann ein Widerstand aus solchem Material eingebaut sein.

Das Frequenzsteuerteil 17 umfaßt auch noch eine Subtraktionsstufe 22, die vom temperaturkompensierten niederfrequenten Signal $S_{NF}$ einen programmierbaren festen Wert (z.B. zwischen 80 bis 90 % des Mittelwertes des niederfrequenten Signals) subtrahiert. Dadurch wird ein für die Steuerung unspezifischer Signalanteil (Offset) eliminiert.

**Patentansprüche**

1. Vorrichtung zur Impedanzmessung an Körpergeweben mit einer Signalquelle (2,12) zum Einprägen eines elektrischen Signals (V ,I ) in das Körpergewebe, einer Einrichtung (7,8,13) zum Erfassen eines Impedanzsignals ($S_I$) aus dem Körpergewebe in Abhängigkeit vom eingeprägten elektrischen Signal (V ,I ) und einer Auswerteeinrichtung (9) zur Auswertung des Impedanzsignals ($S_I$) im Sinne der Heraustrennung der dem Leitwert ($\sigma_R$) entsprechenden niederfrequenten Signalanteile ($S_{NF}$), die einen Signalausgang (10) für die herausgetrennten Signalanteile ($S_{NF}$) umfaßt, **dadurch gekennzeichnet,** daß vom Signalausgang (10) der Auswerteeinrichtung (9) die niederfrequenten Signalanteile als Steuersignal ($S_{NF}$) dem Frequenzsteuerteil (17) eines frequenzgesteuerten Herzschrittmachers (16) zugeführt werden zur Steuerung der Stimulationsfrequenz in dem Sinne, daß bei sich änderndem Leitwert ($\sigma_R$) die Stimulationsfrequenz entsprechend geändert wird.

2. Vorrichtung nach Anspruch 1, **dadurch gekennzeichnet,** daß die Auswerteeinrichtung ein Tiefpaßfilter (9) mit einer oberen Grenzfrequenz im Bereich 0,1 bis 0,4 Hz umfaßt.

3. Vorrichtung nach Anspruch 1 oder 2, **dadurch gekennzeichnet,** daß die Grenzfrequenz des Tiefpaßfilters (9) in Abhängigkeit von der Stimulationsfrequenz in dem Sinne variierbar ist, daß sie mit steigender Stimulationsfrequenz zu höheren Werten und mit sinkender Stimulationsfrequenz entsprechend zu niedrigeren Werten verschoben wird.

4. Vorrichtung nach einem der vorangehenden Ansprüche, **dadurch gekennzeichnet,** daß die Auswerteeinrichtung eine Einrichtung (20) zur Kompensation des Temperatureinflusses auf das niederfrequente Signal ($S_{NF}$) umfaßt.

5. Vorrichtung nach Anspruch 4, **dadurch gekennzeichnet,** daß das Frequenzsteuerteil (17) eine Subtraktionsstufe (22) umfaßt, die vom temperaturkompensierten niederfrequenten Signal ($S_{NF}$) einen programmierbaren festen Wert subtrahiert.

6. Vorrichtung nach Anspruch 5, **dadurch gekennzeichnet,** daß der feste Wert im Bereich von 80 bis 90% des Mittelwertes des niederfrequenten Signals ($S_{NF}$) liegt.

**Claims**

1. Apparatus for measuring the impedance of body tissue, having a signal source (2, 12) for impressing an electrical signal (V, I) into the body tissue, a means (7, 8, 13) for recording an impedance signal ($S_I$) from the body tissue as a function of the impressed electrical signal (V, I) and an evaluation means (9) for evaluating the impedance signal ($S_I$) by filtering out the low-frequency signal components ($S_{NF}$) corresponding to the conductance ($\sigma_R$), said means containing a signal output (10) for the filtered out signal components ($S_{NF}$), characterised in that the low-frequency signal components are supplied as a control signal ($S_{NF}$) from the signal output (10) of the evaluation means (9) to the frequency control unit (17) of a frequency-controlled heart pacemaker (16) for controlling the stimulation frequency in such a way that the stimulation frequency is changed accordingly when the conductance ($\sigma_R$) changes.

2. Apparatus according to Claim 1, characterised in that the evaluation means contains a low-pass filter (9) with an upper limit frequency in the range of 0.1 to 0.4 Hz.

3. Apparatus according to Claim 1 or 2, characterised in that the limit frequency of the low-pass filter (9) can be varied as a function of the stimulation frequency in such a way that it is shifted to higher values given an increasing stimulation frequency and accordingly to lower values given a decreasing stimulation frequency.

4. Apparatus according to one of the preceding claims, characterised in that the evaluation means contains a means (20) for compensating the influence of temperature on the low-frequency signal ($S_{NF}$).

5. Apparatus according to Claim 4, characterised in that the frequency control unit (17) contains a subtraction stage (22) which subtracts a fixed programmable value from the temperature-compensated low-frequency signal ($S_{NF}$).

6. Apparatus according to Claim 5, characterised in that the fixed value lies in the range of 80 to 90 % of the mean value of the low-frequency signal ($S_{NF}$).

**Revendications**

1. Dispositif pour réaliser la mesure de l'impédance de tissus corporels, comportant une source de signaux (2,12) servant à injecter un signal électrique (V,I) dans le tissu corporel, un dispositif (7,8,13) pour détecter le signal d'impédance ($S_I$) délivré par le tissu corporel, en fonction du signal électrique injecté (V,I), et un dispositif d'évaluation (9) servant à évaluer le signal d'impédance ($S_I$) dans le sens d'une séparation des composantes de signal ($S_{NF}$) à basse fréquence, qui correspondent à la conductance ($S_R$), et comportant une sortie (10) pour les composantes séparées de signal ($S_{NF}$), caractérisé par le fait que la sortie (10) du signal du dispositif d'évaluation (9) envoie les composantes de signal à basse fréquence en tant que signal de commande ($S_{NF}$) à la partie de commande de fréquence (17) d'un stimulateur cardiaque (16) dont la fréquence est commandée, pour commander la fréquence de stimulation de telle sorte que, lorsque la conductance ($S_R$) varie, la fréquence de stimulation varie de façon correspondante.

2. Dispositif suivant la revendication 1, caractérisé par le fait que le dispositif d'évaluation comprend un filtre passe-bas (9) possédant une fréquence limite supérieure située dans la gamme comprise entre 0,1 et 0,4 Hz.

3. Dispositif suivant la revendication 1 ou 2, caractérisé par le fait que la fréquence limite du filtre passe-bas (9) peut être modifiée en fonction de la fréquence de stimulation de telle sorte que, lorsque la fréquence de stimulation augmente, la fréquence limite prend des valeurs accrues et, lorsque la fréquence de stimulation diminue, la fréquence limite est décalée de façon correspondante vers des valeurs plus faibles.

4. Dispositif suivant l'une des revendications précédentes, caractérisé par le fait que le dispositif d'évaluation comprend un dispositif (20) servant à compenser l'influence de la température sur le signal à basse fréquence ($S_{NF}$).

5. Dispositif suivant la revendication 4, caractérisé en ce que la partie (17) de commande de la fréquence comprend un étage soustracteur (22), qui soustrait, une valeur fixe programmable, du signal à basse fréquence ($S_{FN}$) compensé en température.

6. Dispositif suivant la revendication 5, caractérisé par le fait que la valeur fixe est située dans la gamme comprise entre 80 et 90 % de la valeur moyenne du signal à basse fréquence ($S_{NF}$).

FIG 1

FIG 2

FIG 3